Europäisches Patentamt

European Patent Office

Office européen des brevets

(19)

(11) Veröffentlichungsnummer : **0 368 796 B1**

(12)

# EUROPÄISCHE PATENTSCHRIFT

(45) Veröffentlichungstag der Patentschrift :
20.05.92 Patentblatt 92/21

(51) Int. Cl.⁵ : **A61F 2/36**

(21) Anmeldenummer : 89730206.3

(22) Anmeldetag : 13.10.89

(54) **Verfahren zur Herstellung einer Schaftprothese.**

(30) Priorität : 19.10.88 DE 3836040

(43) Veröffentlichungstag der Anmeldung :
16.05.90 Patentblatt 90/20

(45) Bekanntmachung des Hinweises auf die
Patenterteilung :
20.05.92 Patentblatt 92/21

(84) Benannte Vertragsstaaten :
AT BE CH DE ES FR GB GR IT LI NL SE

(56) Entgegenhaltungen :
EP-A- 0 058 745
EP-A- 0 093 869
EP-A- 0 145 939
EP-A- 0 209 516
FR-A- 2 549 718
US-A- 4 623 349

(73) Patentinhaber : MECRON MEDIZINISCHE
PRODUKTE GMBH
Nunsdorfer Ring 23-29
W-1000 Berlin 48 (DE)

(72) Erfinder : **Ahrens, Uwe**
**Nürnberger Strasse 46**
**W-1000 Berlin 30 (DE)**
Erfinder : **Kranz, Curt, Dr.**
**Kufsteiner Strasse 12**
**W-1000 Berlin 62 (DE)**
Erfinder : **Neubert, Olaf**
**Wilhelm-Hauff Strasse 16**
**W-1000 Berlin 41 (DE)**
Erfinder : **Götsche, Paul**
**Schrammstrasse 3**
**W-1000 Berlin 31 (DE)**
Erfinder : **Ploetz, Wiebke**
**Schmiljanstrasse 4**
**W-1000 Berlin 41 (DE)**

(74) Vertreter : **Christiansen, Henning, Dipl.-Ing.**
**Patentanwalt Pacelliallee 43/45**
**W-1000 Berlin 33 (DE)**

## Beschreibung

Die Erfindung betrifft ein Verfahren der im Oberbegriff des Anspruchs 1 angegebenen Art sowie eine nach diesem Verfahren hergestellte Schaftprothese.

Eine derartige Schartprothese u. ein Verfahren in ihrer Herstellungsind aus der US-A-4 623 349 bekannt.

Es sind Schaftprothesen mit in Längsrichtung des Schafts verlaufenden Einrillungen bekannt. So wie bei der Schaft prothese, die in der EP-A2-0 145 939 beschrieben ist, verlaufen die mit bosenförmigen Konturen versehenen Einrillungen auf der Oberfläche in gleichen Abständen zueinander.

Bei der aus o.s. US-A-4 623 349 bekannten Schaftprothese verlaufen die in Richtung zum Schaftinneren weisenden Einrillungen ebenfalls in gleichen Abständen zueinander, wobei sich aber deren Breite in Richtung zur Schaftspitze vergrößert. Bei dem entsprechenden Herstellungsverfahren werden der oder die Abtragungsvorrichtungen in Spuren geführt, die bezogen auf die in bezug auf ihre endgültige Kontur schon endbearbeitete Prothese, auf der so erzeugten Oberfläche in gleichen Abständen verlaufen.

Nachteilig ist dabei, daß hiermit lediglich in die Ebene abwickelbare Formen erzeugt werden können. Auch muß der mit den Einrillungen zu versehende Schaft vor der Einarbeitung der Rillen durch Abtragung in seiner äußeren Grundform vorgefertigt sein.

Der Erfindung liegt die Aufgabe zugrunde, bei einem verfairen der eingangs genannten Gattung die Möglichkeit zu schaffen, auch beliebig räumlich verlaufende Oberflächen. mit Einrillungen zu versehen.

Diese Aufgabe wird mit den kennzeichnenden Merkmalen des Anspruchs 1 gelöst.

Die Erfindung beruht auf der Erkenntnis, daß sich in Schaftrichtung verlaufende Einrillungen, die eine gute Rotationsstabilität und eine unproblematische Wieder-Entfernbarkeit des Schaftes ermöglichen, auch auf räumlich. sich beliebig erweiternde oder in sonstiger Weise urregelmäßig geformte Schäfte anwenden lassen. Besonders vorteilhaft ist dabei, daß der Schaft sich durch Nachsinken nach der Implantation "setzen." 'kann, so daß ein guter Sitz gewährleistet ist.

Die Zahl der bei der Herstellung durchlaufenen Fräsbahnen nimmt bevorzugt gleichmäßig stufenweise in der Weise zu, daß in vorbestimmten Stufungen des Radius in der Mitte zwischen zwei benachbarten Bahnen eine weitere Bahn eingeschoben wird.

Günstig ist insbesondere, daß durch das erfindungsgemäße Verfahren die Herstellung durch Fräsen aus einem Rohling in einem Arbeitsgang erfolgen kann.

Insbesondere ist das erfindungsgemäße Verfahren zur vollautomatischen Herstellung von Schaftprothesen nach vom Patienten abgenommenen, individuellen Daten geeignet.

Dabei ist ein im wesentlichen kegelförmiger oder in anderer Weise sich zur Spitze hin verjüngender Fräser vorgesehen, der auf einer Bahn geführt ist, bei der das spitze Ende des Fräsers auf eine Mittelachse ausgerichtet ist, wobei der Fräser auf einzelnen radialen Bahnen geführt ist, die in tangentialer Richtung durch die Mittelachse festliegen, wobei sich die Zahl der Bahnen zur Mittelachse hin verringert und die Bearbeitung in der Weise erfolgt, daß der Fräser sich auf diesen radialen Bahnen bewegend von außen nach innen bis zur Oberfläche der Prothese in ihrem endbearbeiteten Zustand geführt wird, wobei er beim schichtweisen Abtragen jeweils bei den letzten Abtragungsvorgängen der Oberfläche der Prothese in ihrem endbearbeiteten Zustand folgt. Im axialen Querschnitt gesehen haben die Bahnen jeweils höchstens den Abstand einer Bearbeitungsbreite durch den Fräser.

Der Spitzenwinkel des kegelförmigen Fräsers beträgt bevorzugt im wesentlichen 30°. Die Schnittiefe liegt bei der Bearbeitung bevorzugt in einem Bereich von 0,3 bis 0,5 mm, wenn es sich um Flächen handelt, die nicht spanabtragend nachbearbeitet werden. Zur Beschleunigung der Bearbeitung und in Anwendungsfällen, bei denen die Prothesenteile noch nachbearbeitet werden, kann die Schnittiefe bei einer Bearbeitungsvorstufe zunächst auch ca. 1 mm betragen.

Eine entsprechend dem vorgenannten Verfahren hergestellte Schaftprothese zeichnet sich somit insbesondere dadurch aus, daß die Einrillungen einander dicht benachbart verlaufen und daß die Einrillungen unter Verzweigungen auch über sich erweiternde und insbesondere nicht in die Ebene abwickelbare Oberflächenbereiche verlaufen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 ein Ausführungsbeispiel einer nach dem erfindungsgemäßen Verfahren hergestellten Schaftprothese in Seitenansicht,

Figur 2 ein Schema zur Verdeutlichung des Verfahrens mit der Schnittdarstellung einer Schaftprothese sowie

Figur 2a eine Detaildarstellung zu Figur 2 in Seitenansicht als Abwicklung wiedergegeben.

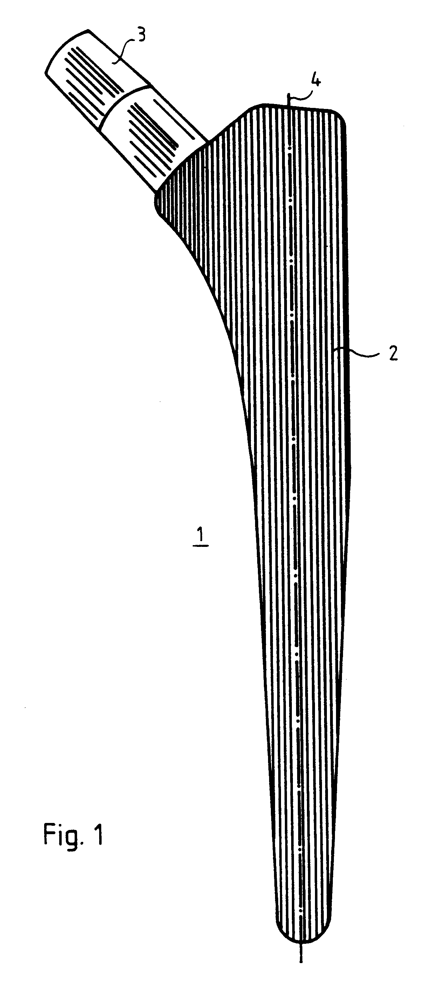

Bei dem in Figur 1 dargestellten Ausführungsbeispiel einer Femurschaftprothese 1 aus Titan, hergestellt nach dem erfindungsgemäßen Verfahren, ist ein Schaft 2 an seinem proximalen Ende mit einem Konus 3 zur Aufnahme der Gelenkkugel versehen. Der Schaft weist Einrillungen auf, die einander dicht benachbart sind und geometrisch einzeln aus jeweils unterschiedlichen Richtungen - sämtlich auf die Achse 4 (strichpunktiert dargestellt) zu projizieren sind.

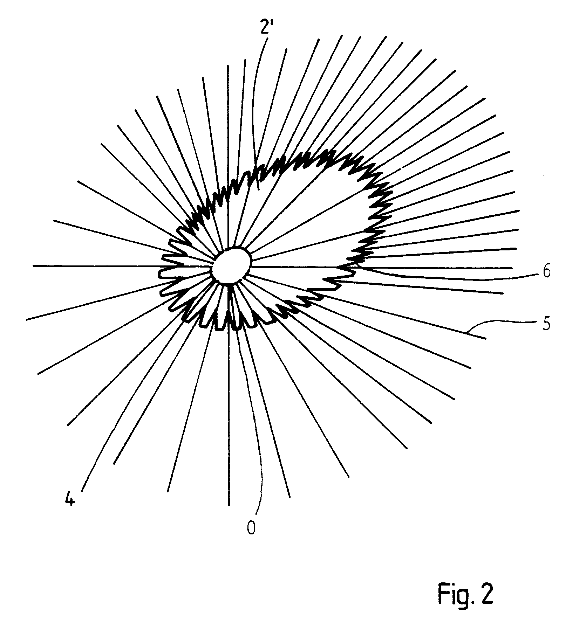

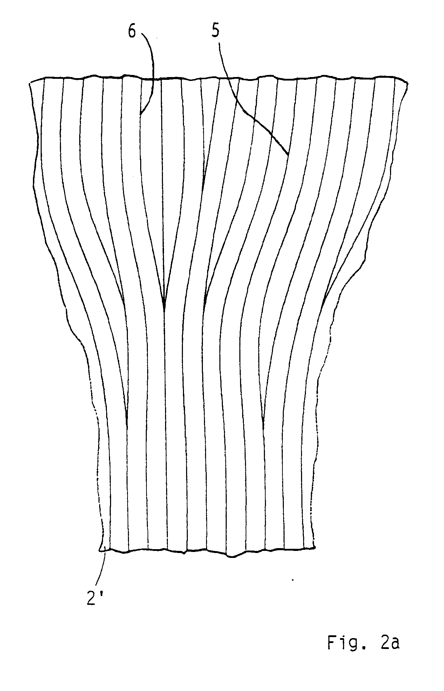

Die Zahl der Rillen nimmt mit zunehmendem Abstand von der Achse 4 zu, wobei in Bereichen, deren Oberfläche zu der Achse 4 geneigt verlaufen, sich in Richtung der Erweiterung die Anzahl der Rillen vermehren. Die Einrillungen können dabei unter Verzweigung über nicht in die Ebene abwickelbare Oberflächenbereiche verlaufen. Derartige Verzweigungen befinden sich insbesondere im bogenförmig zum Konus 3 hin verlaufenden Bereich des Schafts und sind in der weiter unten zu beschreibenden vergrößerten Detaildarstellung in der Abwicklung gemäß Figur 2a im einzelnen wiedergegeben.

Bei der Darstellung in Figur 2 ist anhand des Schnitts eines anderen Schafts 2' erkennbar, daß die Einrillungen der Oberfläche einen im wesentlichen keilförmigen Querschnitt aufweisen, wobei die Einrillungen jeweils in Ebenen verlaufen, die sich in der gemeinsamen Mittelachse 4 schneiden. Die Spitzen der keilförmigen Einrillungen weisen auf diese Mittelachse und die Anzahl der auf einen Winkelbereich mit Scheitelpunkt auf der Mittelachse entfallenden Einrillungen nimmt mit zunehmendem Abstand der Oberfläche von der Mittelachse zu, so daß die Einrillungen auf der Schaftoberfläche einander dicht benachbart verlaufen.

Die nach Art einer Kompaßrose um die senkrecht zur Zeichenebene gerichtete Achse 4 herum angeordneten Geraden, von denen sich ein Teil im Spurpunkt der Achse 4 schneidet, geben die Projektion der Ebenen wieder, die die Linien, auf denen sich der Fräser auf seinem senkrecht zur Zeichenebene gerichteten Weg bewegen kann, enthalten. Die Zahl der möglichen Wege vermehrt sich in radialer Entfernung von der Achse 4, wobei die Zahl der Fräsbahnen gleichmäßig stufenweise in der Weise zunimmt, daß in vorbestimmten Stufungen des Radius in der Mitte zwischen zwei benachbarten Geraden eine weitere Gerade eingeschoben wird, so daß der Abstand zweier Fräsbahnen deren Breite nicht überschreitet. Eine als Beispiel gekennzeichnete Fräsbahn 5 gehört zu einer ersten Gruppe von Fräsbahnen, die einen ausreichenden Abstand haben, um sich bis zu einem inneren, dem Schaft 2' einbeschriebenen Kreis 0 mit einem minimalen zu bearbeitenden Radius - bezogen auf die Mittelachse 4 des Schafts (Figur 1) als Mittelpunkt des Kreises 0 fortgeführt werden zu können.

Mit zunehmendem Abstand vom Kreis 0 wird der Abstand der Fräsbahnen größer, so daß weitere Fräsbahnen eingeschoben werden, um eine gleichmäßige Dichte der Einrillungen auf der Prothesenoberfläche zu erhalten. Als Beispiel einer solchen Fräsbahn aus einer Gruppe von gleichartigen Fräsbahnen ist die Bahn 6 in Figur 2 gekennzeichnet.

In Figur 2a ist ein vergrößerter Ausschnitt als Abwicklung der Schaftoberfläche 2' wiedergegeben. Während die (nicht dargestellte) Achse des Schafts parallel zur Darstellungsebene verläuft, erstreckt sich die in der Draufsicht dargestellte Außenoberfläche - in Richtung von unten nach oben - mit zunehmender Wölbung auf den Betrachter zu. Die Zahl der Fräsbahnen (beispielsweise 5), welche bereits im unteren Abschnitt vorhanden waren, vermehrt sich durch zusätzlich eingefügte (beispielsweise 6) die aus den ursprünglich vorhandenen abzweigen.

Bei dem Verfahren zur Herstellung der Schaftprothese verlaufen damit die Fräsbahnen für die Einrillungen jeweils in Ebenen, die sich in einer gemeinsamen Mittelachse schneiden. Die Spitzen der im Querschnitt keilförmigen Fräsbahnen weisen auf diese Mittelachse und die Anzahl der auf einen Winkelbereich mit Scheitelpunkt auf der Mittelachse entfallenden Fräsbahnen nimmt mit zunehmendem Abstand der Oberfläche von der Mittelachse zu, so daß die Fräsbahnen einander dicht benachbart verlaufen. Das Fräsen erfolgt mittels eines keilförmigen Fräsers aus einem Prothesenrohling, der noch nicht die Konturen der fertigen Prothese (ohne Einrillungen) aufweist. Es erfolgt also ein vollständiger Oberflächenabtrag. Die Zahl der Fräsbahnen nimmt bevorzugt gleichmäßig stufenweise in der Weise zu, daß in vorbestimmten Stufungen des Radius in der Mitte zwischen zwei benachbarten Bahnen eine weitere Bahn eingeschoben wird. Der Spitzenwinkel des kegelförmigen Fräsers beträgt im wesentlichen 30°.

Dieses Verfahren eignet sich insbesondere zur vollautomatischen Herstellung nach vom Patienten abgeleiteten Daten, beispielsweise aus dem Computer-Tomogramm.

Der Fräser bewegt sich beim Herausarbeiten der Schaftform aus dem Rohling beim aufeinanderfolgenden Durchlaufen der Fräsbahnen auf dem Weg von einer Fräsbahn zu einer benachbarten auf einer Spiralbahn in Richtung auf die Achse oder auf konzentrischen Kreisen mit zunehmend verringertem Radius bzw. auf Teilen derartiger Bahnen. Ist der Bereich der bleibenden Kontur des Schafts erreicht, wird der Weg entsprechend pendelnd auf der freien Weglänge zwischen den Bereichen der bleibenden Kontur des Schafts fortgesetzt.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen. Dazu gehört insbesondere auch, daß die Achse nicht geradlinig, sondern leicht gebogen oder auch verwunden verläuft, wobei die Form dieser Achse

EP 0 368 796 B1

bevorzugt an den Weg des Schafts beim Einführen in den Knochen angepaßt ist.

**Patentansprüche**

1. Verfahren zur Herstellung einer Schaftprothese mit durch Abtragung eingearbeiteten, in Richtung zum Schaftinneren weisenden Einrillungen,
**dadurch gekennzeichnet ,**
daß die Abtragung durch Fräsen erfolgt, wobei
die Fräsbahnen jeweils in Ebenen verlaufen, die sich in einer gemeinsamen Mittelachse schneiden,
die Fräsbahnen im Querschnitt keilförmig sind und ihre Spitzen auf diese Mittelachse weisen,
die Anzahl der auf einen Winkelbereich mit Scheitelpunkt auf der Mittelachse entfallenden Fräsbahnen mit zunehmendem Abstand der Oberfläche von der Mittelachse zunimmt und
die Fräsbahnen einander unmittelbar benachbart verlaufen, derart, daß das Fräsen mit einem keilförmigen Fräser aus einem Prothesenrohling unter vollständigem Oberflächenabtrag erfolgt.

2. Verfahren nach Anspruch 1, **dadurch gekennzeichnet,** daß die Zahl der Fräsbahnen mit dem Radius linear zunimmt.

3. Verfahren nach einem der vorangehenden Ansprüche, **dadurch gekennzeichnet,** daß die Zahl der Fräsbahnen gleichmäßig stufenweise in der Weise zunimmt, daß in vorbestimmten Stufungen des zunehmenden Radius zwischen zwei benachbarten Bahnen eine weitere Bahn eingefügt wird.

4. Verfahren nach einem der vorangehenden Ansprüche, **dadurch gekennzeichnet,** daß der Spitzenwinkel des kegelförmigen Fräsers im wesentlichen 30° beträgt.

5. Verfahren nach einem der vorangehenden Ansprüche, **gekennzeichnet durch** die vollautomatische Herstellung nach vom Patienten abgeleiteten Daten.

6. Verfahren nach einem der vorangehenden Ansprüche, **dadurch gekennzeichnet,** daß der Fräser beim Herausarbeiten der Schaftform aus dem Rohling sich beim aufeinanderfolgenden Durchlaufen der Fräsbahnen auf dem Weg von einer Fräsbahn zu einer benachbarten in Richtung auf die Achse auf einer Spiralbahn oder auf konzentrischen Kreisen mit zunehmend verringertem Radius oder Teilen davon bewegt bis der Bereich der verbleibenden Kontur des Schafts erreicht ist.

7. Schaftprothese, hergestellt durch ein Verfahren nach einem der vorangehenden Ansprüche, bei dem die Oberfläche in Richtung zum Schaftinneren weisende Einrillungen aufweist,
dadurch **gekennzeichnet ,**
daß die Einrillungen jeweils in Ebenen verlaufen, die sich in einer gemeinsamen Mittelachse (4) schneiden,
daß die Einrillungen im Querschnitt keilförmig und ihre Spitzen auf diese Mittelachse (4) weisen,
daß die Anzahl der auf einen Winkelbereich mit Scheitelpunkt auf der Mittelachse (4) entfallenden Einrillungen mit zunehmendem Abstand der Oberfläche von der Mittelachse (4) zunimmt und
daß die Einrillungen einander unmittelbar benachbart verlaufen, derart, daß die Einrillungen unter Verzweigungen über sich erweiternde, und insbesondere nicht in eine Ebene abwickelbare Oberflächenbereiche verlaufen.

8. Schaftprothese nach Anspruch 7, **dadurch gekennzeichnet,** daß die Zahl der Einrillungen mit dem Radius linear zunimmt.

9. Schaftprothese nach einem Ansprüche 7 oder 8, **dadurch gekennzeichnet,** daß die Zahl der Fräsbahnen gleichmäßig stufenweise in der Weise zunimmt, daß in vorbestimmten Stufungen des zunehmenden Radius zwischen zwei benachbarten Bahnen eine weitere Bahn eingefügt ist.

10. Schaftprothese nach einem der Ansprüche 7 bis 9, **dadurch gekennzeichnet,** daß der Spitzenwinkel des kegelförmigen Fräsers im wesentlichen 30° beträgt.

11. Schaftprothese nach einem der Ansprüche 7 bis 10, **dadurch gekennzeichnet,** daß die Tiefe der Einrillungen zwischen 0,3 und 0,6 Millimetern beträgt.

**Claims**

1. Method for producing a shaft prosthesis having grooves which are formed by material removal and which point in the direction of the interior of the shaft,
characterised in that
the material removal is carried out by milling, and
the milling paths in each case extend in planes which intersect in a common centre axis,

4

the milling paths are wedge-shaped in cross-section and their tips point towards this centre axis,

the number of milling paths assigned to an angular range with their vertex towards the centre axis increases as the distance of the surface from the centre axis increases, and

the milling paths extend directly adjacent to one another in such a way that the milling with a wedge-shaped milling cutter is carried out on a prosthesis blank with complete surface removal.

2. Method according to Claim 1, characterised in that the number of milling paths increases linearly with the radius.

3. Method according to one of the preceding claims, characterised in that the number of milling paths increases uniformly in a stepped manner in such a way that, at predetermined stages of the increasing radius, a further path is introduced between two adjacent paths.

4. Method according to one of the preceding claims, characterised in that the point angle of the conical milling cutter is essentially 30°.

5. Method according to one of the preceding claims, characterised by the fully automatic production on the basis of data derived from the patient.

6. Method according to one of the preceding claims, characterised in that, when working the shaft shape from the blank, the milling cutter, upon consecutive passage through the milling paths, moves on the way from one milling path to an adjacent one in the direction of the axis on a spiral path or on concentric circles of increasingly reduced radius or parts thereof, until the area of the remaining contour of the shaft is reached.

7. Shaft prosthesis, produced by a method according to one of the preceding claims, in which the surface has grooves pointing in the direction of the interior of the shaft,
characterised in that

the grooves in each case extend in planes which intersect in a common centre axis (4),

the grooves are wedge-shaped in cross-section and their tips point towards this centre axis (4),

the number of grooves assigned to an angular range with their vertex towards the centre axis (4) increases as the distance of the surface from the centre axis (4) increases, and

the grooves extend directly adjacent to one another in such a way that the grooves extend, with branching, over surface areas which widen and, in particular, cannot be unwound into one plane.

8. Shaft prosthesis according to Claim 7, characterised in that the number of grooves increases linearly with the radius.

9. Shaft prosthesis according to one of Claims 7 or 8, characterised in that the number of milling paths increases uniformly in a stepped manner in such a way that, at predetermined stages of the increasing radius, a further path is introduced between two adjacent paths.

10. Shaft prosthesis according to one of Claims 7 to 9, characterised in that the point angle of the conical milling cutter is essentially 30°.

11. Shaft prosthesis according to one of Claims 7 to 10, characterised in that the depth of the grooves is between 0.3 and 0.6 millimetres.

**Revendications**

1. Procédé de fabrication d'une prothèse à tige comportant des rainurages pratiqués par enlèvement, dirigés vers l'intérieur de la tige,
caractérisé en ce que
l'enlèvement s'effectue par fraisage,

les voies de fraisage s'étendant chacune dans des plans qui se coupent sur un axe médian commun,

les voies de fraisage ayant une section transversale en cône et leurs pointes étant dirigées vers cet axe médian,

le nombre des voies de fraisage, se situant sur une zone angulaire avec sommet sur l'axe médian, augmentant en même temps qu'augmente la distance de la surface par rapport à l'axe médian et

les voies de fraisage étant directement adjacentes les unes aux autres de telle sorte que le fraisage s'effectue avec une fraise conique à partir d'une ébauche de prothèse, par enlèvement total de la surface.

2. Procédé selon la revendication 1, caractérisé en ce que le nombre des voies de fraisage augmente linéairement avec le rayon.

3. Procédé selon l'une des revendications précédentes, caractérisé en ce que le nombre des voies de fraisage augmente progressivement et régulièrement de manière que dans des gradations prédéterminées du rayon croissant, une autre voie s'insère entre deux voies adjacentes.

4. Procédé selon l'une des revendications précédentes, caractérisé en ce que l'angle de sommet de la fraise conique est à peu près égal à 30°.

5. Procédé selon l'une des revendications précédentes, caractérisé par la fabrication entièrement automatique d'après des données dérivées du patient.

6. Procédé selon l'une des revendications précédentes, caractérisé en ce que la fraise se déplace, lors de l'usinage de la forme de la tige dans l'ébauche, au cours de la traversée successive des voies de fraisage, sur le parcours menant d'une voie de fraisage à une voie de fraisage adjacente, en direction de l'axe, sur une voie en spirale ou sur des cercles concentriques de rayon décroissant ou sur des parties de celles-ci, jusqu'à ce que la zone du contour restant de la tige soit atteinte.

7. Prothèse à tige, fabriquée par un procédé selon l'une des revendications précédentes, dans laquelle la surface présente des rainurages dirigés vers l'intérieur de la tige,

caractérisée en ce que

les rainurages s'étendent chacun dans des plans qui se coupent sur un axe médian (4) commun,

les rainurages ont une section transversale en cône et leurs pointes sont dirigées vers cet axe médian (4),

le nombre des rainurages se situant sur une zone angulaire avec sommet sur l'axe médian (4), augmente en même temps qu'augmente la distance de la surface par rapport à l'axe médian (4) et

en ce que les rainurages sont directement adjacents les uns aux autres de telle sorte que les rainurages s'étendent en formant des ramifications sur des zones de surface s'élargissant, et en particulier ne pouvant pas se développer dans un plan.

8. Prothèse à tige selon la revendication 7, caractérisée en ce que le nombre des rainurages augmente linéairement avec le rayon.

9. Prothèse à tige selon l'une des revendications 7 ou 8, caractérisée en ce que le nombre des voies de fraisage augmente régulièrement et progressivement de manière que dans des gradations prédéterminées du rayon croissant, une autre voie s'insère entre deux voies adjacentes.

10. Prothèse à tige selon l'une des revendications 7 à 9, caractérisée en ce que l'angle de sommet de la fraise conique est à peu près ègal à 30°.

11. Prothèse à tige selon l'une des revendications 7 à 10, caractérisée en ce que la profondeur des rainurages est comprise entre 0,3 et 0,6 mm.

Fig. 1

Fig. 2

6    5

2'

Fig. 2a